# EUROPEAN PATENT APPLICATION

(11) **EP 4 804 565 A1**
(43) Date of publication of application: **09.09.2026**
(21) Application number: 26162373.0
(22) Date of filing: 04.03.2026
(51) Int. Cl.: H04R 25/00

(54) **SYSTEMS AND METHODS FOR FACILITATING IMPLEMENTATION OF A SUBCLINICAL HEARING LOSS OPERATING MODE BY A HEARING DEVICE**

(30) Priority: 05.03.2025 US 202519071350
(71) Applicant: Sonova AG, 8712 Stäfa (CH)
(72) Inventor: Courtois, Gilles, 1720 Corminboeuf (CH)

(57) **Abstract**

An exemplary system comprising a memory that stores instructions and a processor communicatively coupled to the memory and configured to execute the instructions to perform a process. The process may comprise selecting a subclinical hearing loss operating mode (304) for a hearing device; and automatically applying, to the hearing device (202) and based on the subclinical hearing loss operating mode (304), a plurality of operating parameters (308-1, 308-2, ..., 308-N) optimized for users with subclinical hearing loss.

## Description

### BACKGROUND INFORMATION

Hearing devices (e.g., hearing aids) are used to improve the hearing capability and/or communication capability of users of the hearing devices. Such hearing devices are configured to process a received input sound signal (e.g., ambient sound) and provide the processed input sound signal to the user (e.g., by way of a receiver (e.g., a speaker) placed in the user's ear canal or at any other suitable location).

Conventional hearing devices are typically fit to users through the use of hearing device fitting software. Such software relies on the provision of an audiogram and some user information (e.g., age, experience with hearing devices, etc.) to select a suitable earpiece (e.g., open domes) and calculate the insertion gain and sound processing features default strength (e.g., denoiser, frequency compression, etc.) for a selected hearing device model. This approach is well suited for clinical hearing losses, in which the audiogram shows elevated hearing thresholds that must be compensated by amplification. However, this approach fails to address subclinical hearing losses.

Individuals that have subclinical hearing loss typically have a normal to near normal audiogram that does not cause the hearing device fitting software to recommend amplification. As a result, individuals with subclinical hearing losses who take the initiative to seek the help of a hearing care professional are most of the time recommended to wait until their hearing experience degrades before coming back to the clinic, despite their typically having difficulty understanding speech in certain complex listening environments (e.g., noisy backgrounds, loud environments, multi-talker scenarios, etc.). In cases in which a hearing care professional may decide to fit a hearing aid to subclinical hearing loss, a rather delicate and elaborate tuning followed by a testing phase and a subsequent fine-tuning of the hearing aid can be required to make the device compatible with the specific needs of the individual. Accordingly, there remains room to improve the manner in which hearing devices are used to facilitate sound perception for individuals with subclinical hearing loss.

### DESCRIPTION OF THE INVENTION

It is an objective of the invention to avoid at least one of the above mentioned disadvantages and to provide for an automatized configuration of hearing device parameters which are optimized for subclinical loss, in particular to improve speech understanding in complex listening environments while reducing the need for extensive manual tuning.

This objective is achieved by the subject-matter of the independent claims. Further exemplary embodiments are evident from the dependent claims and the following description.

A first aspect of the invention relates to a system comprising a memory that stores instructions; and a processor communicatively coupled to the memory and configured to execute the instructions to perform a process comprising:
selecting a subclinical hearing loss operating mode for a hearing device in which the hearing device is customized to a user having subclinical hearing loss; and
automatically applying, to the hearing device and based on the subclinical hearing loss operating mode, a plurality of operating parameters optimized for users with subclinical hearing loss.

According to an embodiment, the method further comprises determining that the user of the hearing device has subclinical hearing loss, wherein the subclinical hearing loss operating mode is selected based on the determined subclinical hearing loss.

According to an embodiment, the determining that the user of the hearing device has subclinical hearing loss is based on at least one of prior knowledge from additional users, feedback provided by the user, or suprathreshold measurements of hearing impairment.

According to an embodiment, the automatically applying of the plurality of operating parameters is independent of an audiogram of the user.

According to an embodiment, the automatically applying of the plurality of operating parameters includes setting one or more of a gain parameter, a compression parameter, a maximum power output parameter, or a signal processing parameter for the hearing device based on the subclinical hearing loss of the user.

According to an embodiment, the process further comprises
obtaining an audiogram;
calculating, based on the audiogram, an insertion gain;
determining, when the subclinical hearing loss operating mode is selected, whether the insertion gain is smaller than a minimal gain, and, when the insertion gain is smaller than the minimal gain, setting the insertion gain to a predetermined floor insertion gain, wherein the automatically applying the plurality of operating parameters optimized for users with subclinical hearing loss comprises applying the insertion gain.

According to an embodiment, the operating parameters included in the plurality of operating parameters are optimized for speech perception in noise for the users with subclinical hearing loss.

According to an embodiment, the process further comprises determining a type of hearing device for the user based on the subclinical hearing loss of the user.

According to an embodiment, the automatically applying of the plurality of operating parameters to the hearing device includes selectively applying at least some operating parameters included in the plurality of operating parameters based on a context of an environment of the user.

According to an embodiment, the process further comprises
determining that the user of a hearing device has clinical hearing loss;
selecting, based on the clinical hearing loss of the user, a clinical hearing loss operating mode for the hearing device;
and automatically applying, to the hearing device and based on the clinical hearing loss operating mode, a plurality of operating parameters optimized for users with clinical hearing loss.

According to an embodiment, the process further comprises
obtaining an audiogram; and
calculating, based on the audiogram, an insertion gain,
   wherein, when the subclinical hearing loss operating mode is selected, the method further comprises determining whether the insertion gain is smaller than a minimal gain, and, when the insertion gain is smaller than the minimal gain, setting the insertion gain to a predetermined floor insertion gain, wherein the automatically applying the plurality of operating parameters optimized for users with subclinical hearing loss comprises applying the insertion gain,
   and, when the clinical hearing loss operating mode is selected, the insertion gain is maintained according to the calculation based on the audiogram, wherein the automatically applying the plurality of operating parameters optimized for users with clinical hearing loss comprises applying the insertion gain.

According to an embodiment, the process further comprises
obtaining an audiogram; and
providing, in the hearing device, one or more speech enhancement features, wherein a strength of the speech enhancement provided by the speech enhancement features is adjustable,
   wherein, when the subclinical hearing loss operating mode is selected, the automatically applying the plurality of operating parameters optimized for users with subclinical hearing loss comprises adjusting the strength of one or more of the speech enhancement features to a predetermined strength independent from the audiogram, in particular a maximum strength;
   and, when the clinical hearing loss operating mode is selected, the automatically applying the plurality of operating parameters optimized for users with clinical hearing loss comprises adjusting the strength of one or more of the speech enhancement features to a strength depending on the audiogram.

Some exemplary speech enhancement features may comprise, e.g., a beamforming algorithm, a noise cancellation algorithm, and/or a machine learning algorithm, in particular a neural network (NN), such as a deep neural network (DNN) which may be trained to separate one or more speech signals from noise in an audio signal.

According to an embodiment, the process further comprises
providing a plurality of clinical sound processing patterns attributed to different input sound classifications associated with a sound environment of a user, wherein the clinical sound processing patterns are optimized for sound processing in the clinical hearing loss operating mode;
providing a plurality of subclinical sound processing patterns attributed to the different input sound classifications, wherein the subclinical sound processing patterns are optimized for sound processing in the subclinical hearing loss operating mode;
selecting, when the clinical hearing loss operating mode is selected, one or more of the clinical sound processing patterns, wherein the automatically applying the plurality of operating parameters optimized for users with clinical hearing loss comprises directing the hearing device to process, when the hearing device determines the input sound classification during operation of the hearing device, the sound in accordance with the one or more selected clinical sound processing patterns attributed to the input sound classification determined by the hearing device; and
selecting, when the subclinical hearing loss operating mode is selected, one or more of the subclinical sound processing patterns, wherein the automatically applying the plurality of operating parameters optimized for users with subclinical hearing loss comprises directing the hearing device to process, when the hearing device determines the input sound classification during operation of the hearing device, the sound in accordance with the one or more selected subclinical sound processing patterns attributed to the input sound classification determined by the hearing device.

The hearing device may include a sound classifier for determining the input sound classification based on an audio signal received by the hearing device. E.g., the audio signal may be based on sound detected by one or more microphones included in the hearing device. In some examples, the one or more sound processing patterns, in particular clinical and/or subclinical sound processing patterns, may be implemented as different sound processing programs, in particular actuators, that may be executed by the hearing device.

According to an embodiment, the automatically applying of the plurality of operating parameters to the hearing device includes
selecting an input sound classification associated with sound in an environment of a user;
selecting, based on the input sound classification, a first subclinical hearing loss sound processing pattern from a plurality of subclinical hearing loss sound processing patterns; and
directing the hearing device to process the sound in accordance with the first subclinical hearing loss sound processing pattern.

According to an embodiment, the first subclinical hearing loss sound processing pattern is optimized for speech perception in noise for users with subclinical hearing loss.

According to an embodiment, the automatically applying of the plurality of operating parameters to the hearing device includes
selecting an additional input sound classification associated with sound in an environment of a user;
selecting, based on the additional input sound classification, a second subclinical hearing loss sound processing pattern from the plurality of subclinical hearing loss sound processing patterns; and
directing the hearing device to process the sound in accordance with the second subclinical hearing loss sound processing pattern instead of the first subclinical hearing loss sound processing pattern.

According to an embodiment, the second subclinical hearing loss sound processing pattern is optimized for a context in which the user does not require speech enhancement.

According to an embodiment, the system is a hearing device management system. In some examples, the hearing device management system may be implemented as a fitting system used for a fitting of the hearing device. E.g., the fitting system may implemented as a fitting computer and/or an application on a mobile device, e.g., a smartphone. The fitting system may be implemented to be operated by a third-person, e.g., a health care professional, and/or to be operated by the user, e.g., in a self-fitting procedure.

In the context of the disclosed technology, clinical hearing loss may refer to a hearing impairment that is detectable with standard clinical testing, such as pure-tone audiometry, in the form of hearing thresholds exceeding a predefined limit, e.g., above 25 dB HL. In some examples, clinical hearing loss may imply that an average of the hearing thresholds over standard audiometric frequencies (e.g., between 250 Hz and 8000 Hz) exceeds the predefined threshold limit.

Subclinical hearing loss may refer to a hearing impairment, e.g., a self-reported hearing impairment by the user, other than clinical hearing loss, in particular a hearing impairment that cannot be identified, e.g., diagnosed, based on the hearing thresholds exceeding a predefined limit attributed to clinical hearing loss. In particular, subclinical hearing loss may refer to a hidden hearing loss, at which the hearing thresholds may be smaller or equal to the predefined limit at all standard audiometric frequencies (e.g., between 250 Hz and 8000 Hz), and/or minimal hearing loss, at which the hearing thresholds may exceed the predefined limit characteristic at some frequencies only, for example, within a higher frequency range (e.g., between 2000 Hz and 8000 Hz), wherein the average of the hearing thresholds at all standard audiometric frequencies may be smaller or equal to the predefined limit.

A further aspect of the invention relates to a method comprising:
determining, by a hearing device management system, that a user of a hearing device has subclinical hearing loss;
selecting, by the hearing device management system and based on the subclinical hearing loss of the user, a subclinical hearing loss operating mode for the hearing device; and
automatically applying, by the hearing device management system to the hearing device and based on the subclinical hearing loss operating mode, a plurality of operating parameters optimized for users with subclinical hearing loss.

According to an embodiment, the determining that the user of the hearing device has subclinical hearing loss is based on at least one of prior knowledge from additional users, feedback provided by the user, or suprathreshold measurements of hearing impairment.

According to an embodiment, the automatically applying of the plurality of operating parameters is independent of an audiogram of the user.

According to an embodiment, the automatically applying of the plurality of operating parameters includes setting one or more of a gain parameter, a compression parameter, a maximum power output parameter, or a signal processing parameter for the hearing device based on the subclinical hearing loss of the user.

According to an embodiment, operating parameters included in the plurality of operating parameters are optimized for speech perception in noise for the users with subclinical hearing loss.

According to an embodiment, the method further comprises determining a type of hearing device for the user based on the subclinical hearing loss of the user.

According to an embodiment, the automatically applying of the plurality of operating parameters to the hearing device includes selectively applying at least some operating parameters included in the plurality of operating parameters based on a context of an environment of the user.

According to an embodiment, the method comprises
selecting (e.g., determining) by the hearing device management system (for example, while the hearing device is operating according to the subclinical hearing loss operating mode), an input sound classification associated with sound in an environment of a user;
selecting, by the hearing device management system and based on the input sound classification, a first subclinical hearing loss sound processing pattern from a plurality of subclinical hearing loss sound processing patterns; and
directing, by the hearing device management system, the hearing device to process the sound in accordance with the first subclinical hearing loss sound processing pattern.

According to an embodiment, the first subclinical hearing loss sound processing pattern is optimized for speech perception in noise for users with subclinical hearing loss.

According to an embodiment, the first subclinical hearing loss sound processing pattern includes setting one or more of a gain parameter, a compression parameter, a maximum power output parameter, or a signal processing parameter for the hearing device based on the subclinical hearing loss of the user.

According to an embodiment, the method comprises:
selecting (e.g., determining), by the hearing device management system (for example, while the hearing device is operating according to the subclinical hearing loss operating mode), an additional input sound classification associated with the sound in the environment of the user;
selecting, by the hearing device management system and based on the additional input sound classification, a second subclinical hearing loss sound processing pattern from the plurality of subclinical hearing loss sound processing patterns; and
directing, by the hearing device management system, the hearing device to process the sound in accordance with the second subclinical hearing loss sound processing pattern instead of the first subclinical hearing loss sound processing pattern.

According to an embodiment, the second subclinical hearing loss sound processing pattern is optimized for a context in which the user does not require speech enhancement.

According to an embodiment, the hearing device is communicatively connected to an external device that provides audio content to the hearing device (e.g., a remote microphone, in particular a table microphone); and the first subclinical hearing loss sound processing pattern is optimized for the audio content provided by the external device.

It has to be understood that features of the method as described in the above and in the following may be features of the system, in particular the hearing device management system, and vice versa.

These and other aspects of the invention will be apparent from and elucidated with reference to the embodiments described hereinafter.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIG. 1 illustrates an exemplary hearing device management system that may be implemented according to principles described herein.
FIG. 2 illustrates an exemplary implementation of the hearing device management system of FIG. 1 according to principles described herein.
FIGS. 3-6 illustrate exemplary flow diagrams that may be implemented according to principles described herein.
FIGS. 7-8 illustrate exemplary methods according to principles described herein.
FIG. 9 illustrates an exemplary computing device according to principles described herein.

### DETAILED DESCRIPTION OF EXAMPLARY EMBODIMENTS

Systems and methods for facilitating implementation of a subclinical hearing loss operating mode by a hearing device are described herein. As will be described in more detail below, an exemplary system may comprise a memory storing instructions and a processor communicatively coupled to the memory and configured to execute the instructions to perform a process. The process may comprise determining that a user of a hearing device has subclinical hearing loss; selecting, based on the subclinical hearing loss of the user, a subclinical hearing loss operating mode for the hearing device; and automatically applying, to the hearing device and based on the subclinical hearing loss operating mode, a plurality of operating parameters optimized for users with subclinical hearing loss.

By using systems and methods such as those described herein, it may be possible to facilitate hearing device users that have subclinical hearing loss more easily perceive sound in certain listening environments. For example, systems and methods such as those described herein may be optimized for hearing device users that have difficultly perceiving speech in noisy environments but that have little to no difficulty perceiving speech in quiet environments. To that end, systems and methods such as those described herein may be configured to implement a subclinical hearing loss operating mode to process sound in certain environments for such users. In so doing, it is possible to provide a meaningful improvement in hearing to a historically underserved group of individuals that experience subclinical hearing losses. Other benefits of the systems and methods described herein will be made apparent herein.

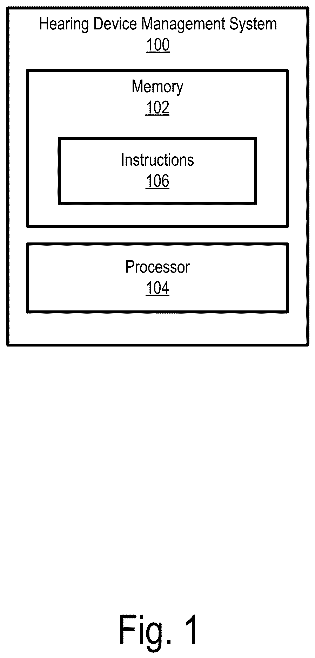

FIG. 1 illustrates an exemplary hearing system 100 ("system 100") that may be implemented according to principles described herein. As shown, system 100 may include, without limitation, a memory 102 and a processor 104 selectively and communicatively coupled to one another. Memory 102 and processor 104 may each include or be implemented by hardware and/or software components (e.g., processors, memories, communication interfaces, instructions stored in memory for execution by the processors, etc.). In some examples, memory 102 and/or processor 104 may be implemented by any suitable computing device such as described herein. In other examples, memory 102 and/or processor 104 may be distributed between multiple devices and/or multiple locations as may serve a particular implementation. Illustrative implementations of system 100 are described herein.

Memory 102 may maintain (e.g., store) executable data used by processor 104 to perform any of the operations described herein. For example, memory 102 may store instructions 106 that may be executed by processor 104 to perform any of the operations described herein. Instructions 106 may be implemented by any suitable application, software, code, and/or other executable data instance.

Memory 102 may also maintain any data received, generated, managed, used, and/or transmitted by processor 104. Memory 102 may store any other suitable data as may serve a particular implementation. For example, memory 102 may store hearing loss profile data, user preference data, setting data, acoustic parameter data, machine learning data, input sound classification data, subclinical hearing loss sound processing pattern data, graphical user interface content, and/or any other suitable data.

Processor 104 may be configured to perform (e.g., execute instructions 106 stored in memory 102 to perform) various processing operations associated with implementing a subclinical hearing loss operating mode. For example, processor 104 may perform one or more operations described herein to determine that a user of a hearing device has subclinical hearing loss, select, based on the subclinical hearing loss of the user, a subclinical hearing loss operating mode for the hearing device, and automatically apply, to the hearing device and based on the subclinical hearing loss operating mode, a plurality of operating parameters optimized for users with subclinical hearing loss. These and other operations that may be performed by processor 104 are described herein.

As used herein, a "hearing device" may be implemented by any device or combination of devices configured to provide or enhance hearing to a user. For example, a hearing device may be implemented by a hearing aid configured to amplify audio content to a recipient, a sound processor included in a cochlear implant system configured to apply electrical stimulation representative of audio content to a recipient, a sound processor included in a stimulation system configured to apply electrical and acoustic stimulation to a recipient, or any other suitable hearing prosthesis. In some examples, a hearing device may be implemented by a behind-the-ear ("BTE") housing configured to be worn behind an ear of a user. In some examples, a hearing device may be implemented by an in-the-ear ("ITE") component configured to at least partially be inserted within an ear canal of a user. In some examples, a hearing device may include a combination of an ITE component, a BTE housing, and/or any other suitable component.

In certain examples, hearing devices such as those described herein may be implemented as part of a binaural hearing system. Such a binaural hearing system may include a first hearing device associated with a first ear of a user and a second hearing device associated with a second ear of a user. In such examples, the hearing devices may each be implemented by any type of hearing device configured to provide or enhance hearing to a user of a binaural hearing system. In some examples, the hearing devices in a binaural system may be of the same type. For example, the hearing devices may each be hearing aid devices. In certain alternative examples, the hearing devices may be of a different type. For example, a first hearing device may be a hearing aid and a second hearing device may be a sound processor included in a cochlear implant system.

In some examples, a hearing device may additionally or alternatively include earbuds, headphones, hearables (e.g., smart headphones), over-the-counter (OTC) hearing aids, active noise cancelling (ANC) devices, and/or any other suitable device that may be used to facilitate a user perceiving sound.

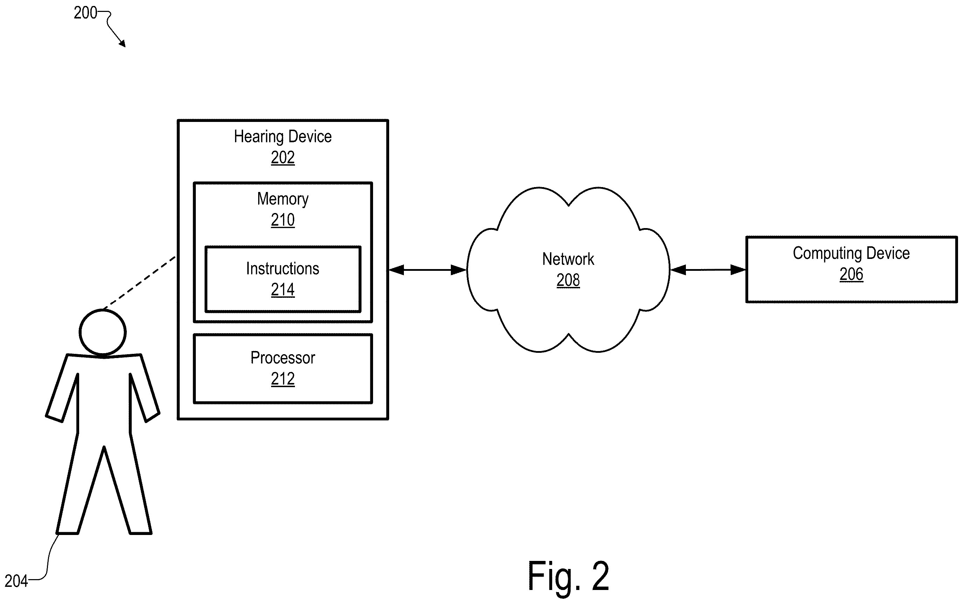

System 100 may be implemented in any suitable manner. For example, system 100 may be implemented by a hearing device and/or a computing device that is communicatively coupled in any suitable manner to the hearing device. To illustrate an example, FIG. 2 shows an exemplary implementation 200 in which system 100 may be provided in certain implementations. As shown in FIG. 2, implementation 200 includes a hearing device 202 that is associated with a user 204 and that is communicatively coupled to a computing device 206 by way of a network 208.

Hearing device 202 may correspond to any suitable type of hearing device such as described herein. Hearing device 202 may include, without limitation, a memory 210 and a processor 212 selectively and communicatively coupled to one another. Memory 210 and processor 212 may each include or be implemented by hardware and/or software components (e.g., processors, memories, communication interfaces, instructions stored in memory for execution by the processors, etc.). In some examples, memory 210 and processor 212 may be housed within or form part of an ITE component. In some examples, memory 210 and processor 212 may be located separately from ITE component (e.g., in a BTE housing). In some alternative examples, memory 210 and processor 212 may be distributed between multiple devices (e.g., multiple hearing devices in a binaural hearing system) and/or multiple locations as may serve a particular implementation.

Memory 210 may maintain (e.g., store) executable data used by processor 212 to perform any of the operations associated with hearing device 202. For example, memory 210 may store instructions 214 that may be executed by processor 212 to perform any of the operations associated with hearing device 202 assisting a user in hearing. Instructions 214 may be implemented by any suitable application, software, code, and/or other executable data instance.

Memory 210 may also maintain any data received, generated, managed, used, and/or transmitted by processor 212. For example, memory 210 may maintain any suitable data associated with a hearing loss profile of a user, input sound classifications, subclinical hearing loss sound processing patterns, machine learning algorithms, and/or hearing device function data. Memory 210 may maintain additional or alternative data in other implementations.

Processor 212 is configured to perform any suitable processing operation that may be associated with hearing device 202. For example, when hearing device 202 is implemented by a hearing aid device, such processing operations may include monitoring ambient sound and/or representing sound to user 204 via an in-ear receiver. Processor 212 may be implemented by any suitable combination of hardware and software. In certain examples, processor 218 may correspond to or otherwise include one or more deep neural network ("DNN") chips configured to perform any suitable machine learning operation such as described herein.

Hearing device 202 may include further components as may serve a particular implementation. In some examples, hearing device 202 may further include one or more microphones for detecting an input sound and/or a radio receiver for receiving radio waves representative of input sound detected by a remote microphone. Hearing device 202 may further include an audio output unit for outputting the audio content to the user, which may be implemented, e.g., as a receiver of a hearing aid, or a loudspeaker of an earbud.

User 204 may correspond to any individual that is a user of a hearing device and that experiences subclinical hearing loss. As used herein, "subclinical hearing loss" may refer to an auditory disfunction that encompasses hidden hearing loss and minimal hearing loss. Hidden hearing loss may be characterized by (1) a self-reported hearing difficulty associated with speech perception in noise and (2) a normal tonal audiometry in both ears (e.g., hearing thresholds that are better than or equal to 25 dB HL at all standard audiometric frequencies between 250 Hz and 8000 Hz). Minimal hearing loss may be characterized by (1) a self-reported hearing difficultly with speech perception in noise, (2) at least one hearing threshold worse than 25 dB HL at standard audiometric frequencies between 2000 Hz and 8000 Hz in at least one ear, (3) and pure tone averages (0.5-4 kHz) that are better than or equal to 25 dB HL in both ears.

Computing device 206 may include or be implemented by any suitable hardware and/or software components (e.g., processors, memories, communication interfaces, instructions stored in memory for execution by the processors, etc.) and may include any combination of computing devices as may serve a particular implementation. In certain examples, computing device 206 may correspond to a laptop computer, a desktop computer, a tablet computer, and/or any other suitable computing device that may be configured to facilitate implementing a subclinical hearing loss operating mode. In such examples, computing device 206 may be configured to perform any suitable operations such as those described herein to optimize sound processing for user 204 by way of hearing device 202. In certain examples, computing device 206 may be implemented as part of a hearing device fitting system configured to fit hearing device 202 to user 204. In such examples, system 100 may provide one or more graphical user interfaces by way of computing device 202 to facilitate a hearing care professional (e.g., a clinician) fitting hearing device 202 to user 204 during a fitting procedure. In this regard, computing device 206 may be configured to implement any suitable fitting software that is configured to perform any of the operations described herein to automatically adjust one or more parameters of hearing device 202 based on the subclinical hearing loss of user 204.

Network 208 may include, but is not limited to, one or more wireless networks (Wi-Fi networks), wireless communication networks, mobile telephone networks (e.g., cellular telephone networks), mobile phone data networks, broadband networks, narrowband networks, the Internet, local area networks, wide area networks, and any other networks capable of carrying data and/or communications signals between hearing device 202 and computing device 206. In certain examples, network 208 may be implemented by a Bluetooth protocol (e.g., Bluetooth Classic, Bluetooth Low Energy ("LE"), etc.) and/or any other suitable communication protocol to facilitate communications between hearing device 202 and computing device 206. Communications between hearing device 202, computing device 206, and any other device/system may be transported using any one of the above-listed networks, or any combination or sub-combination of the above-listed networks.

System 100 may be implemented by computing device 206 or hearing device 202. Alternatively, system 100 may be distributed across computing device 206 and hearing device 202, or distributed across computing device 206, hearing device 202, and/or any other suitable computing system/device.

As mentioned, conventional hearing device fitting systems rely on an audiogram of a user to determine which hearing device operating parameters (e.g., how much gain to apply) to adjust to fit a hearing device to a particular user. However, with subclinical hearing loss, the audiogram of the user may indicate that the user has normal to near normal hearing. As such, a conventional hearing device fitting system may not recommend a hearing device for a user with subclinical hearing loss even though the user has difficulty perceiving sound and/or interpreting perceived sound in certain environments. To address these issues, system 100 may be configured to facilitate implementation of a subclinical hearing loss operating mode that is configured specifically for users with subclinical hearing loss.

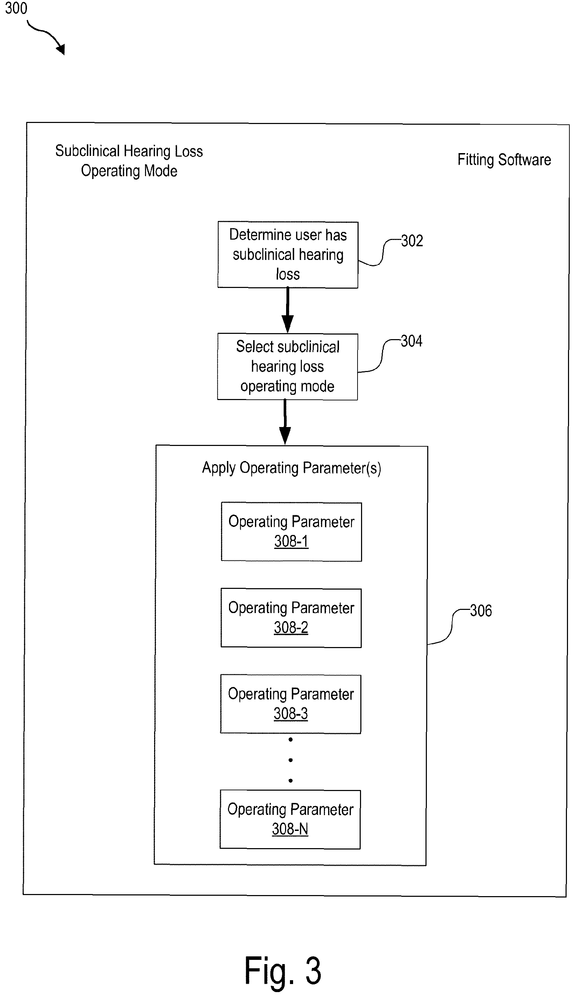

FIG. 3 shows an exemplary flow diagram 300 that may be implemented by system 100 (e.g., computer device 206) in implementing a subclinical hearing loss operating mode. As shown in FIG. 3, system 100 may determine whether a user has subclinical hearing loss at operation 302. This may be accomplished in any suitable manner. For example, system 100 may determine whether a user has subclinical hearing loss based on prior knowledge from additional users, feedback provided by the user, and/or suprathreshold measurements of hearing impairment of the user. In such examples, the prior knowledge from additional users may include any information regarding what types and/or frequencies of sounds are difficult for such users, what listening environment contexts are difficult for such users, etc.

The feedback provided by the user may include any information that a user may provide regarding their difficulty in perceiving sound, implementation preferences, and/or any other suitable information. In certain examples, such feedback may be provided in response to one more questionnaires that may be provided to a user. For example, standardized questionnaires such as the 12-item Speech, Spatial and Qualities of Hearing Scale (SSQ12), the Hearing Handicap Inventory for Adults (HHIA), and/or the Tinnitus and Hearing Survey (THS) may be used to collect such feedback. Such questionnaires facilitate quantifying the difficulties and/or needs of individuals with subclinical hearing losses, which is of particular interest given that the audiogram of such individuals typically does not provide as valuable insights as it does for conventional (clinical) hearing losses. Such questionnaires may include questions like "does a hearing problem cause you difficulty when attending a party?," "does a hearing problem cause you difficulty hearing/understanding coworkers, clients, or customers?," etc. Depending on the answers provided by the user to such questions, system 100 may determine whether the user has subclinical hearing loss. For example, if the user responded "Yes" and "Sometimes," respectively, to these example inquiries, system 100 may determine that the user has subclinical hearing loss.

The suprathreshold measurements of hearing impairment may indicate hidden hearing loss and/or very mild/mild hearing loss. Such suprathreshold measurements may be determined in any suitable manner. For example, an individual spectral sensitivity measurement may be useful to provide an estimation regarding particular damage to inner and/or outer hair cells of user 202. Such damage may not be discernible from the hearing thresholds of user 202 indicated in an audiogram because neighboring auditory filters may mask the damage in the frequency region. However, a relatively higher modulation detection threshold than is normal in the frequency region may be indicative of the damage and may be used to determine whether user 202 has hidden hearing loss.

In certain examples, an audiogram of user 202 may be used to confirm that user 202 has subclinical hearing loss. For example, if the audiogram of user 202 closely corresponds to the audiogram of a normal hearing person (e.g., has hearing thresholds that are better than or equal to 25 dB HL at all standard audiometric frequencies between 250 Hz and 8000 Hz), but user 202 nonetheless has hearing problems (e.g., suprathreshold hearing impairments at thresholds above the thresholds indicated in the audiogram), system 100 may determine that user 202 has subclinical hearing loss.

At operation 304, system 100 may select a subclinical hearing loss operating mode for the hearing device. In some examples, the subclinical hearing loss operating mode for the hearing device may be selected based on the determined subclinical hearing loss of the user. This may be accomplished in any suitable manner. For example, system 100 may select the subclinical hearing loss operating mode for the hearing device during a fitting procedure performed at the office of a hearing care professional. The subclinical hearing loss operating mode for the hearing device may also be selected based on a control command which may be received via a data interface. E.g., such a control command may be input by the user via a user interface, e.g. to manually select the subclinical hearing loss operating mode for the hearing device, and/or during a fitting procedure via a data interface with a fitting computer. In some examples, operation 302 may then be omitted to be executed by system 100 and/or may be executed by another means and/or system.

At operation 306, system 100 may automatically apply, to the hearing device and based on the subclinical hearing loss operating mode, a plurality of operating parameters 308 (e.g., operating parameters 308-1 through 308-N) optimized for users with subclinical hearing loss. As used herein, the expression "automatically" means that an operation or a series of operations (e.g., selecting a subclinical hearing device operating mode, switching subclinical hearing loss sound processing patterns, etc.) are performed without requiring any further input from user 204. For example, based on the satisfaction of the predefined condition, system 100 may direct hearing device 202 to implement one or more of operating parameters 308 to facilitate user 204 perceiving sound in certain listening environments.

Operating parameters 308 may include any suitable operating parameter or combination of operating parameters that may facilitate a user (e.g., user 204) with subclinical hearing loss perceiving sound. For example, operating parameters 308 may include a gain parameter, a compression parameter, a maximum power output parameter, a signal processing parameter for the hearing device based on the subclinical hearing loss of the user, and/or any other suitable parameter. For example, operating parameters 308 may comprise an insertion gain to be applied by the hearing device to provide for improved speech intelligibility (e.g., in accordance with a Speech Intelligibility Index-based curve). For example, operating parameters 308 may comprise a strength of one or more speech enhancement features which may be adjusted at operation 356, e.g., according to a predetermined strength, in particular a maximum strength. For example, operating parameters 308 may comprise a plurality of subclinical sound processing patterns optimized for sound processing in the subclinical hearing loss operating mode, which may be selected at operation 306 to be applied by the hearing device.

System 100 may be configured to apply one or more of parameters 308 in any suitable manner to facilitate users with subclinical hearing loss perceiving sound. In certain examples, system 100 may apply one or more of operating parameters 308 in a manner that is independent of an audiogram of user 204. For example, a gain parameter can be environment-dependent and may be automatically applied by system 100 independent of the audiogram of user 204.

In certain examples, system 100 may automatically apply a dedicated insertion gain prescription as part of a subclinical hearing loss operating mode. The insertion gain may ensure that the speech enhancement processing performed by the hearing device is made available in situations where this is needed, such as for speech in noise situations. Contrary to conventional (clinical) hearing losses, the insertion gain may be independent or partially independent of the audiogram of a user such that a user with normal audiometric thresholds (e.g., a user with hidden hearing loss) would still be provided with mild gain to benefit from the hearing device processing. For a user with a minimal loss, system 100 may set insertion gain independent of the audiogram at frequencies where the hearing thresholds are normal and implement an audiogram-based insertion gain at frequencies where the user has clinical hearing losses.

In certain examples, the insertion gain applied as part of the subclinical hearing loss operating mode may only be positive for certain listening environments. For example, for relatively quieter environments, the insertion gain may be set to negative values to ensure that the user is not bothered by hearing device artifacts (e.g., circuit noise, comb filtering, etc.) that may be perceived at low levels. In certain examples, the insertion gain may be constant over frequency to favor sound naturalness or emphasize frequencies contributing the most to speech intelligibility as defined, for example, by the Speech Intelligibility Index.

In certain examples, the insertion gain selected based on the subclinical hearing loss operating mode may be linear (i.e., no compression) to avoid a reduction of the gain at loud levels, as typically prescribed for conventional (clinical) hearing losses. This is because individuals with subclinical hearing losses typically encounter more hearing difficulties at high levels than at low levels and compression may therefore be counterproductive as a rehabilitation method. Also, linear amplification is the most natural type of amplification and avoids compression-based artefacts such as the pumping effect. On the other end, compression may be preferred by hearing device users to mitigate loudness discomfort, which would typically occur in users with hyperacusis. In such cases, the gain reduction may be frequency-independent (i.e., the gain is equally reduced at all frequencies) or may follow the natural spectrum modification of speech at high levels (Lombard effect).

In certain examples, the insertion gain and compression may be defined by a third-party fitting formula designed for individuals with subclinical hearing losses.

With respect to maximum power output, system 100 may prescribe maximum power output values to use for the subclinical hearing loss operating mode that are relatively lower than those defined for conventional (clinical) hearing losses. This may ensure that the use of hearing devices for users with subclinical hearing loss does not damage the auditory system of those users and result in degradation of hearing performance in the long term.

With respect to signal processing features, system 100 may set signal processing features to a strong strength by default in speech in noise environments/programs. This may include any suitable signal processing feature such as single channel or multichannel noise reduction, static or adaptive monaural or binaural beamformers, and/or deterministic or Al-based methods, the availability of which depends on the selected hearing device. System 100 may also apply specific internal parameter sets and algorithmic architectures (e.g., specific latency, bandwidth, etc.) that would be different from the parametrization used for conventional (clinical) hearing losses. In programs intended for calm and/or non-communicative situations, the features may be turned off for users with subclinical hearing loss. Alternatively, signal processing features aiming at reducing low-level noises (like circuit noise) may be set to a strong strength for calm and/or non-communicative programs/environments.

In certain examples, one or more of parameters 308 selected by system 100 may be optimized to facilitate connectivity of a hearing device to an external device (e.g., a smart phone, a tablet computer, etc.) that provides audio content to the hearing device. In such examples, one or more of parameters 308 may be optimized to facilitate a user with subclinical hearing loss perceive the audio content provided by way of the external device in different listening environments.

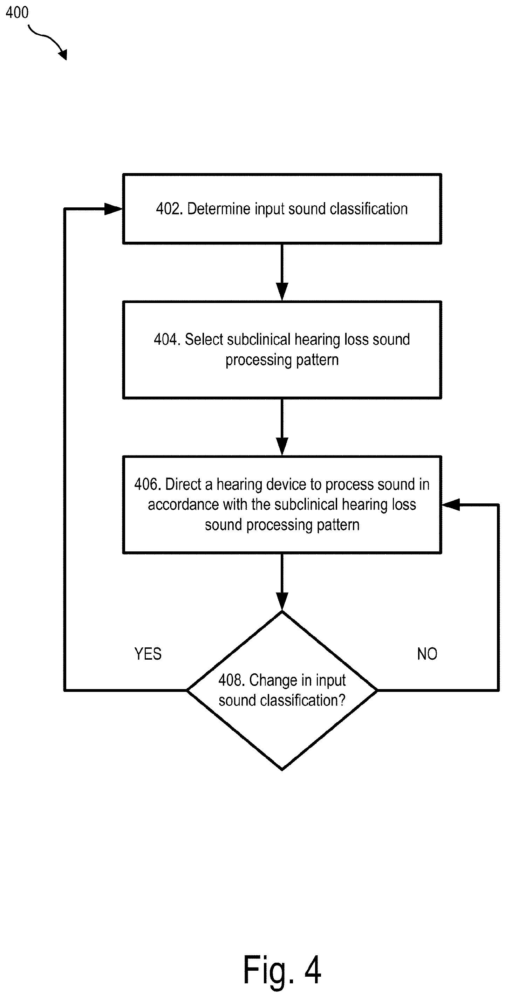

In certain examples, the automatically applying of the plurality of operating parameters to the hearing device may include selectively applying at least some operating parameters included in the plurality of operating parameters based on a context of an environment of the user. In some examples, the context of the environment may correspond to a classification of a sound in the environment, e.g., a current acoustic scene such as noise, speech in noise, speech in quiet, music, traffic sound, etc. To illustrate, FIG. 4 shows an exemplary flow diagram 400 that depicts a flow of operations that may be performed by system 100 (e.g., hearing device 202 and/or computing device 206) to implement a subclinical hearing loss operating mode. As shown in FIG. 4, at operation 402, system 100 may determine, while hearing device 202 is operating according to a subclinical hearing loss operating mode, an input sound classification associated with sound in an environment of user 204. The input sound classification may correspond to any suitable classification that may be associated with sound in an environment where user 204 is located. In certain examples, there may be a plurality of different input sound classifications that may be associated with sound in an environment. For example, there may be a first input sound classification, a second input sound classification, a third input sound classification, and so forth. Each input sound classification may be associated with a different sound situation that may be experienced by user 204 during use of hearing device 202. For example, a first input sound classification may correspond to a speech-in-noise classification, a second input sound classification may correspond to a quiet environment classification, a third input sound classification may correspond to a multi-talkers classification, a fourth input sound classification may correspond to a loud environment classification, and so forth.

System 100 may determine the input sound classification in any suitable manner. For example, system 100 may use a microphone of hearing device 202 to detect sound in the environment surrounding user 204 during use of hearing device 202. Based on the detected sound, system 100 may determine whether the input sound classification corresponds, for example, to a speech-in-noise classification, a quiet environment classification, or any other suitable type of input sound classification such as those described herein.

In some examples, the sound classification may be determined by a classifier based on the input sound representative of a current sound environment. For instance, the classifier may be implemented in hearing device 202 as a sound classification routine executed by processor 212. In some examples, different classes may be attributed to different audio processing programs (e.g., actuators) executed by hearing device 202, e.g., by processor 212. E.g., one or more of the different audio processing programs attributed the current environment may be (automatically) executed based on the sound classification so as to provide for the output audio content optimized for the current environment.

In determining the input sound classification, system 100 may be trained to distinguish different sounds and/or speech from background noise. In certain examples, system 100 may be trained specifically for the needs of user 204 by artificial intelligence using any suitable machine learning methodology.

At operation 404, system 100 may select, based on the input sound classification, a first subclinical hearing loss sound processing pattern from a plurality of subclinical hearing loss sound processing patterns. Each subclinical hearing loss sound processing pattern may define specific sound processing parameters and/or settings that are adapted for a specific input sound classification. For example, the first subclinical hearing loss sound processing pattern may be specific to a speech in noise listening context. In such an example, the first subclinical hearing loss sound processing pattern may be optimized for speech perception in noise for users with subclinical hearing loss. As such, the first subclinical hearing loss sound processing pattern may define one or more specific settings/parameters that facilitate user 204 perceiving speech while in a noisy environment such as at a party or a crowded restaurant.

The plurality of subclinical hearing loss sound processing patterns may include any suitable number of subclinical hearing loss sound processing patterns as may serve a particular implementation. For example, the plurality of subclinical hearing loss sound processing parameters may include a first subclinical hearing loss sound processing pattern, a second subclinical hearing loss sound processing pattern, a third subclinical hearing loss sound processing pattern, and so forth.

At operation 406, system 100 may direct hearing device 202 to process sound in accordance with the subclinical hearing loss sound processing pattern selected at operation 404. This may be accomplished in any suitable manner. For example, system 100 may provide any suitable instruction signal to hearing device 202 that instructs hearing device 202 to begin processing sound during use in accordance with the selected subclinical hearing loss sound processing pattern.

During use of hearing device 202, the sound environment may change such that the subclinical hearing loss sound processing pattern selected at operation 404 may not be optimal for the current sound environment. Accordingly, at operation 408, system 100 may determine whether there has been a change in the input sound classification or whether a change in the input sound classification is about to occur. If the answer at operation 408 is "NO," the flow may return to operation 406 and hearing device 202 may continue to operate in accordance with the subclinical hearing loss sound processing pattern selected at operation 404. If the answer at operation 408 is "YES," the flow may return to operation 402 where system 100 may determine an additional input sound classification associated with the sound in the environment of user 204. System 100 may then repeat operation 404 and select a second subclinical hearing loss sound processing program from the plurality of subclinical hearing loss sound processing programs. System 100 may then direct hearing device 202 to process sound in accordance with the second subclinical hearing loss sound processing pattern instead of the first subclinical hearing loss sound processing pattern. To illustrate an example, the second subclinical hearing loss sound processing pattern selected by system 100 may be optimized for a context in which user 204 does not require sound enhancement, in particular speech enhancement. An example of this may be when user 204 moves from a noisy environment to a quiet environment. In such examples, the second subclinical hearing loss sound processing pattern may result in hearing device 202 not providing sound enhancement because user 204 does not need help perceiving speech in a quiet environment. System 100 may repeat operations 402-408 any suitable number of times to implement a subclinical hearing loss operating mode.

In certain examples, system 100 may select a hearing device model or a type of hearing device for a user based on the subclinical hearing loss of the user. In such examples, system 100 may select a hearing device model or type of hearing device that is the most appropriate to the needs and/or preferences of individuals with subclinical hearing loss. Examples of such models/types may include ones with limited insertion gain (which typically excludes power models), visually discreet models (e.g., receiver in canal (RIC) hearing devices), products configured to implement advanced noise reduction features, etc. System 100 may select which parameters 308 to apply to hearing device 204 based on the type and/or model of hearing device selected.

In certain examples, system 100 may select the type of earpiece to use for users based on their preferences. For example, system 100 may select open or vented domes as a default type of earpiece to use depending on the intended use of the user. Vented domes may be preferentially selected as they give access to the highest speech enhancement performance due to the passive attenuation of direct sound. However, vented domes may generate some occlusion effects (own voice resonance), which may be cumbersome for users in the long term. As such, system 100 may select vented domes if the user only intends to situationally wear them (e.g., only in noisy environments). Open dome earpieces may provide relatively better listening comfort with no occlusion effects, at the cost of slightly reduced performance. As such, system 100 may select an open dome type in instances where the user intends to continually wear the hearing device. As another example, system 100 may select an ANC device, e.g., an ANC earpiece, as a compromise between a desired degree of direct sound attenuation, at least for lower frequencies, and a low occlusion effect.

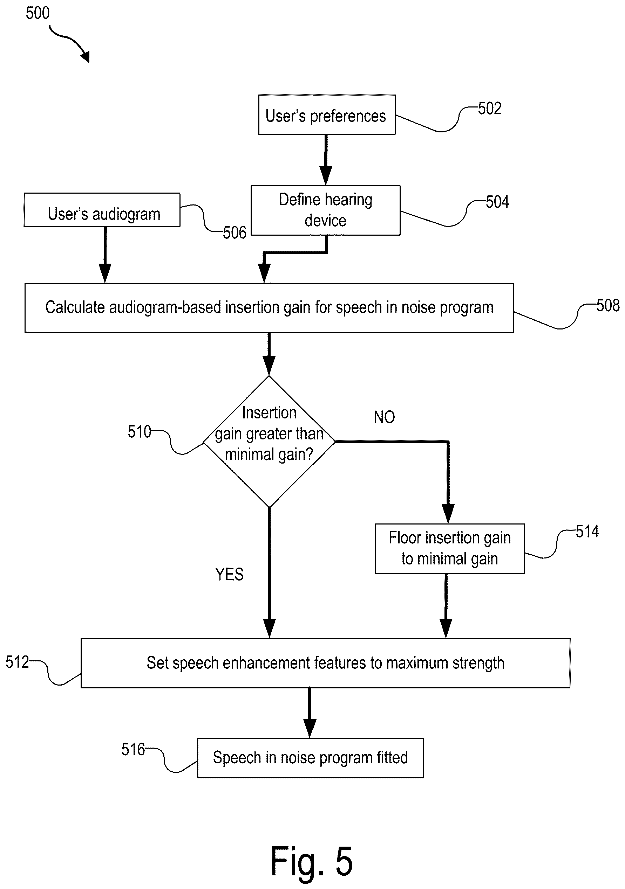

FIG. 5 shows an exemplary flow diagram 500 with additional operations that may be performed by system 100 to facilitate implementing a subclinical hearing loss operating mode. At operation 502, system 100 may access information associated with a user's preferences associated with a hearing device. Such information may include preferences regarding visibility of the hearing device, intended use of the hearing device, and/or any other suitable preference information. Such preference information may be accessed from any suitable source such as one or more questionnaires that may be filled out by the user.

At operation 504, system 100 may define which hearing device is best suited for the user based on the preference information accessed at operation 502. For example, system 100 may determine that an open dome RIC hearing device is best suited for the user based on the user's desire for the hearing device to be minimally visible and worn throughout the day.

At operation 506, system 100 may access an audiogram of the user in any suitable manner from any suitable source.

At operation 508, system 100 may calculate an audiogram-based insertion gain for a speech in noise program to be implemented by the particular hearing device defined at operation 504.

At operation 510, system 100 may determine whether the insertion gain is greater than a minimal gain. If the answer at operation 510 is "YES", system 100 may set, at operation 512, speech enhancement features to maximum strength, e.g., for a speech in noise context. For example, the speech enhancement features may comprise beamforming and/or noise cancellation and/or speech separation which may be performed by a machine learning algorithm, in particular a neural network (NN), such as a deep neural network (DNN).

If the answer at operation 510 is "NO", system 100 may set the insertion gain, as calculated at 508, to a floor insertion gain at operation 514. The floor insertion gain can be provided as any predefined gain, for example, a gain which is suitable to emphasize speech content (e.g., a Speech Intelligibility Index-based curve). In particular, the floor insertion gain may be set to the minimal gain at operation 514. It is understood that the floor insertion gain is independent of the audiogram for speech in noise situations. In non-speech in noise situations, the hearing device may be muted or disabled because no speech enhancement is needed and muting or disabling the hearing device avoids sensitive sound artifacts being presented to the user.

At operation 516, system 100 may conclude that the speech in noise program is fitted to the hearing device for a speech in noise context.

In certain implementations, system 100 may be configured to operate in accordance with a clinical hearing loss operating mode in which an audiogram of user 202 is measured and a gain parameter is fitted to the measured audiogram according to a predetermined prescription rule (e.g., fitting formula). In such examples, system 100 may determine, based on an audiogram indicative of auditory thresholds related to a clinical hearing loss, that user 204 also has clinical hearing loss. Based on the subclinical hearing loss of user 204, system 100 may select a clinical hearing loss operating mode for hearing device 202. Based on the audiogram, system 100 may automatically apply a gain parameter to hearing device 202 based on a prescription rule defining a mapping between the audiogram and the gain parameter.

In certain examples, system 100 may further apply, in a subclinical hearing loss operating mode, a gain parameter that is relatively stronger than a gain parameter that would be applied in the clinical hearing loss operating mode (based on the prescription rule applied on the audiogram) over at least part of a frequency range.

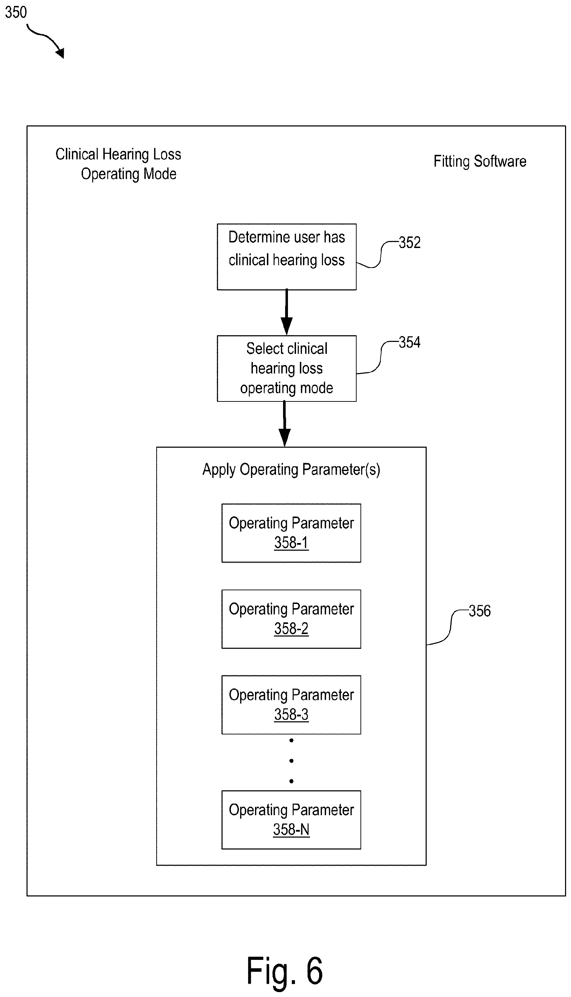

FIG. 6 shows an exemplary flow diagram 350 that may be implemented by system 100 (e.g., computer device 206) in implementing a clinical hearing loss operating mode. As shown in FIG. 6, system 100 may determine whether a user has clinical hearing loss at operation 352. This may be accomplished in any suitable manner. For example, system 100 may obtain an audiogram of the user 204. For example, system 100 may be configured to receive, via a data input, a previously measured audiogram and/or to perform standard clinical testing, such as pure-tone audiometry, to obtain the audiogram and/or perform other diagnostic methods such as speech testing. For example, system 100 may determine that the user has clinical hearing loss based on the hearing thresholds indicated by the audiogram exceeding a predefined limit.

At operation 354, system 100 may select a clinical hearing loss operating mode for the hearing device based on the clinical hearing loss of the user. This may be accomplished in any suitable manner. For example, system 100 may select the clinical hearing loss operating mode for the hearing device during a fitting procedure performed at the office of a hearing care professional.

At operation 356, system 100 may automatically apply, to the hearing device and based on the subclinical hearing loss operating mode, a plurality of operating parameters 358 (e.g., operating parameters 358-1 through 358-N) optimized for users with clinical hearing loss. For example, operating parameters 358 may comprise an insertion gain to be applied by the hearing device to compensate for the clinical hearing loss. The applied insertion gain may then be applied depending on the audiogram, in particular to provide for an adjustment (or fitting) of the insertion gain to the individual hearing thresholds of the user indicated by the audiogram. To this end, the insertion gain may be calculated based on the audiogram. For example, operating parameters 358 may comprise a strength of one or more speech enhancement features which may be adjusted at operation 356, e.g., depending on an audiogram. For example, operating parameters 358 may comprise a plurality of clinical sound processing patterns optimized for sound processing in the clinical hearing loss operating mode, which may be selected at operation 356 to be applied by the hearing device.

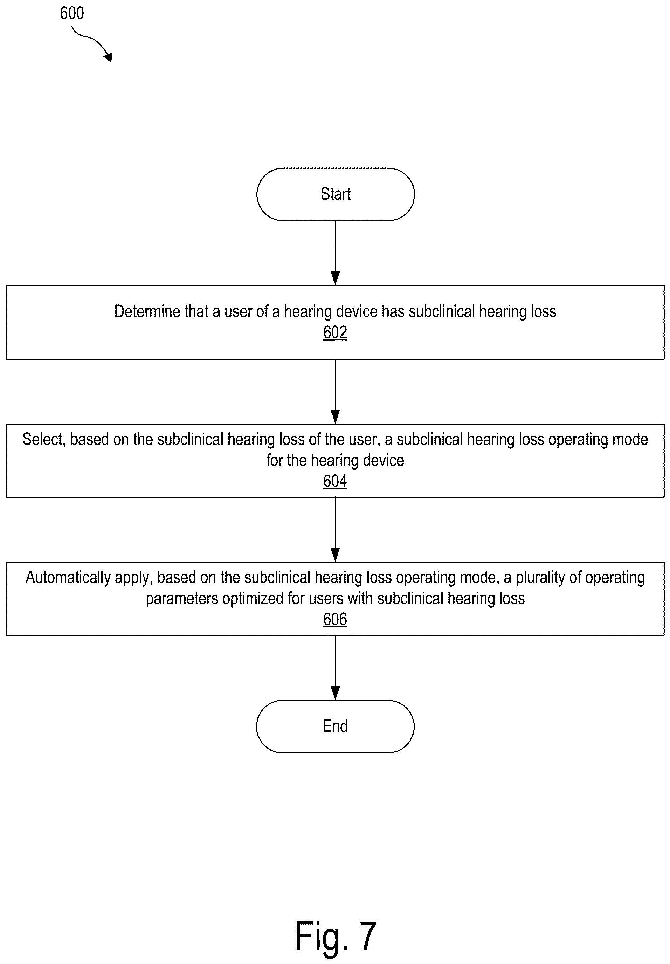

FIG. 7 illustrates an exemplary method 600 for facilitating implementation of a subclinical hearing loss operating mode by a hearing device according to principles described herein. While FIG. 7 illustrates exemplary operations according to one embodiment, other embodiments may omit, add to, reorder, and/or modify any of the operations shown in FIG. 7. One or more of the operations shown in FIG. 6 may be performed by a hearing device such as hearing device 202, a computing device such as computing device 206, an additional computing device communicatively coupled to computing device 206 and/or hearing device 202, any components included therein, and/or any combination or implementation thereof.

At operation 602, a hearing device management system such as hearing device management system 100 may determine that a user of a hearing device has subclinical hearing loss. Operation 602 may be performed in any of the ways described herein.

At operation 604, the hearing device management system may select, based on the subclinical hearing loss of the user, a subclinical hearing loss operating mode for the hearing device. Operation 604 may be performed in any of the ways described herein.

At operation 606, the hearing device management system may automatically apply, based on the subclinical hearing loss operating mode, a plurality of operating parameters optimized for users with subclinical hearing loss. Operation 606 may be performed in any of the ways described herein.

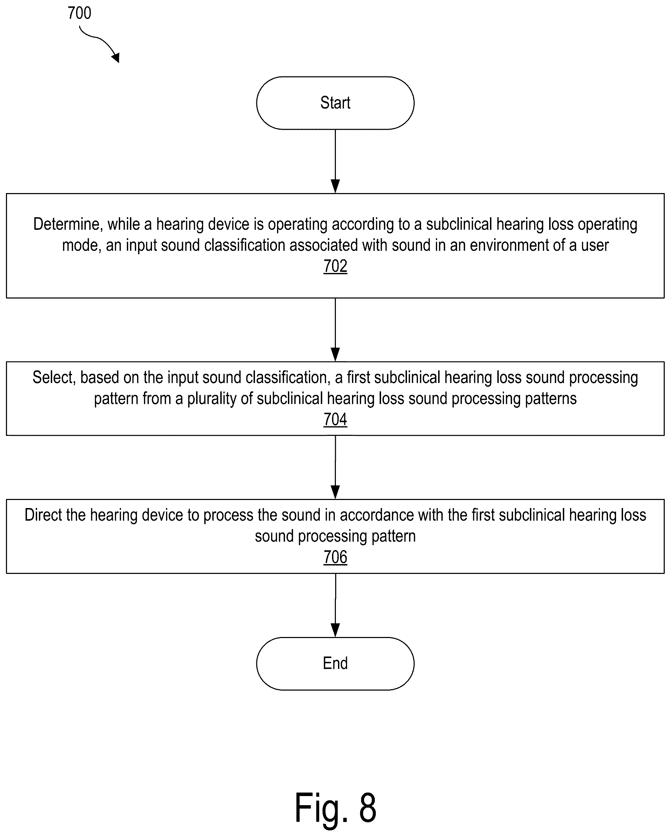

FIG. 8 illustrates another exemplary method 700 for facilitating implementation of a subclinical hearing loss operating mode by a hearing device according to principles described herein. While FIG. 8 illustrates exemplary operations according to one embodiment, other embodiments may omit, add to, reorder, and/or modify any of the operations shown in FIG. 8. One or more of the operations shown in FIG. 7 may be performed by a hearing device such as hearing device 202, a computing device such as computing device 206, an additional computing device communicatively coupled to computing device 206 and/or hearing device 202, any components included therein, and/or any combination or implementation thereof.

At operation 702, a hearing device management system such as hearing device management system 100 may determine, while a hearing device is operating according to a subclinical hearing loss operating mode, an input sound classification associated with sound in an environment of a user. Operation 702 may be performed in any of the ways described herein.

At operation 704, the hearing device management system may select, based on the input sound classification, a first subclinical hearing loss sound processing pattern from a plurality of subclinical hearing loss sound processing patterns. Operation 704 may be performed in any of the ways described herein.

At operation 706, the hearing device management system may direct the hearing device to process the sound in accordance with the first subclinical hearing loss sound processing pattern. Operation 706 may be performed in any of the ways described herein.

In some examples, a computer program product embodied in a non-transitory computer-readable storage medium may be provided. In such examples, the non-transitory computer-readable storage medium may store computer-readable instructions in accordance with the principles described herein. The instructions, when executed by a processor of a computing device, may direct the processor and/or computing device to perform one or more operations, including one or more of the operations described herein. Such instructions may be stored and/or transmitted using any of a variety of known computer-readable media.

A non-transitory computer-readable medium as referred to herein may include any non-transitory storage medium that participates in providing data (e.g., instructions) that may be read and/or executed by a computing device (e.g., by a processor of a computing device). For example, a non-transitory computer-readable medium may include, but is not limited to, any combination of non-volatile storage media and/or volatile storage media. Exemplary non-volatile storage media include, but are not limited to, read-only memory, flash memory, a solid-state drive, a magnetic storage device (e.g., a hard disk, a floppy disk, magnetic tape, etc.), ferroelectric random-access memory ("RAM"), and an optical disc (e.g., a compact disc, a digital video disc, a Blu-ray disc, etc.). Exemplary volatile storage media include, but are not limited to, RAM (e.g., dynamic RAM).

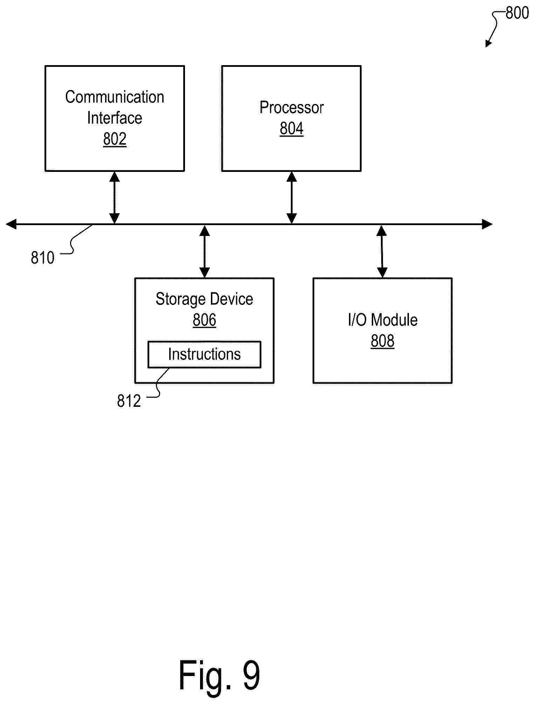

FIG. 9 illustrates an exemplary computing device 800 that may be specifically configured to perform one or more of the processes described herein. As shown in FIG. 8, computing device 800 may include a communication interface 802, a processor 804, a storage device 806, and an input/output ("I/O") module 808 communicatively connected one to another via a communication infrastructure 810. While an exemplary computing device 800 is shown in FIG. 8, the components illustrated in FIG. 8 are not intended to be limiting. Additional or alternative components may be used in other embodiments. Components of computing device 800 shown in FIG. 8 will now be described in additional detail.

Communication interface 802 may be configured to communicate with one or more computing devices. Examples of communication interface 802 include, without limitation, a wired network interface (such as a network interface card), a wireless network interface (such as a wireless network interface card), a modem, an audio/video connection, and any other suitable interface.

Processor 804 generally represents any type or form of processing unit capable of processing data and/or interpreting, executing, and/or directing execution of one or more of the instructions, processes, and/or operations described herein. Processor 804 may perform operations by executing computer-executable instructions 812 (e.g., an application, software, code, and/or other executable data instance) stored in storage device 806.

Storage device 806 may include one or more data storage media, devices, or configurations and may employ any type, form, and combination of data storage media and/or device. For example, storage device 806 may include, but is not limited to, any combination of the non-volatile media and/or volatile media described herein. Electronic data, including data described herein, may be temporarily and/or permanently stored in storage device 806. For example, data representative of computer-executable instructions 812 configured to direct processor 804 to perform any of the operations described herein may be stored within storage device 806. In some examples, data may be arranged in one or more databases residing within storage device 806.

I/O module 808 may include one or more I/O modules configured to receive user input and provide user output. I/O module 808 may include any hardware, firmware, software, or combination thereof supportive of input and output capabilities. For example, I/O module 808 may include hardware and/or software for capturing user input, including, but not limited to, a keyboard or keypad, a touchscreen component (e.g., touchscreen display), a receiver (e.g., an RF or infrared receiver), motion sensors, and/or one or more input buttons.

I/O module 808 may include one or more devices for presenting output to a user, including, but not limited to, a graphics engine, a display (e.g., a display screen), one or more output drivers (e.g., display drivers), one or more audio speakers, and one or more audio drivers. In certain embodiments, I/O module 808 is configured to provide graphical data to a display for presentation to a user. The graphical data may be representative of one or more graphical user interfaces and/or any other graphical content as may serve a particular implementation.

In some examples, any of the systems, hearing devices, computing devices, and/or other components described herein may be implemented by computing device 800. For example, memory 102 and/or memory 210 may be implemented by storage device 806, and processor 104 and/or processor 212 may be implemented by processor 804.

In the preceding description, various exemplary embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense.

## Claims

1. A system comprising:
a memory (102, 210) that stores instructions (106, 214); and
a processor (104, 212) communicatively coupled to the memory (102, 210) and configured to execute the instructions (106, 214) to perform a process comprising:
selecting a subclinical hearing loss operating mode (304) for a hearing device (202) in which the hearing device is customized to a user having subclinical hearing loss; and
automatically applying, to the hearing device (202) and based on the subclinical hearing loss operating mode (304), a plurality of operating parameters (308-1, 308-2, ..., 308-N) optimized for users with subclinical hearing loss.

2. The system of claim 1, wherein the process further comprises
determining that the user of the hearing device (202) has subclinical hearing loss, wherein the subclinical hearing loss operating mode (304) is selected based on the determined subclinical hearing loss.

3. The system of any one of the preceding claims, wherein the automatically applying of the plurality of operating parameters (308-1, 308-2, ..., 308-N) is independent of an audiogram (506) of the user.

4. The system of any one of the preceding claims, wherein the automatically applying of the plurality of operating parameters (308-1, 308-2, ..., 308-N) includes setting one or more of a gain parameter, a compression parameter, a maximum power output parameter, or a signal processing parameter for the hearing device (202) based on the subclinical hearing loss of the user.

5. The system of any one of the preceding claims, wherein the process further comprises
obtaining an audiogram (506);
calculating, based on the audiogram, an insertion gain;
determining, when the subclinical hearing loss operating mode (304) is selected, whether the insertion gain is smaller than a minimal gain, and, when the insertion gain is smaller than the minimal gain, setting the insertion gain to a predetermined floor insertion gain (514), wherein the automatically applying the plurality of operating parameters (308-1, 308-2, ..., 308-N) optimized for users with subclinical hearing loss comprises applying the insertion gain.

6. The system of any one of the preceding claims, wherein the process further comprises determining a type of hearing device (202) for the user based on the subclinical hearing loss of the user.

7. The system of any one of the preceding claims, wherein the process further comprises
determining that the user of a hearing device has clinical hearing loss; selecting, based on the clinical hearing loss of the user, a clinical hearing loss operating mode (354) for the hearing device;
and automatically applying, to the hearing device (202) and based on the clinical hearing loss operating mode, a plurality of operating parameters (358-1, 358-2, ..., 358-N) optimized for users with clinical hearing loss.

8. The system of claim 7, wherein the process further comprises
obtaining an audiogram (506);
calculating, based on the audiogram, an insertion gain (508),
wherein, when the subclinical hearing loss operating mode (304) is selected, the method further comprises determining whether the insertion gain is smaller than a minimal gain, and, when the insertion gain is smaller than the minimal gain, setting the insertion gain (508) to a predetermined floor insertion gain (514), wherein the automatically applying the plurality of operating parameters (308-1, 308-2, ..., 308-N) optimized for users with subclinical hearing loss comprises applying the insertion gain (508);
and, when the clinical hearing loss operating mode (354) is selected, the insertion gain is maintained according to the calculation based on the audiogram, wherein the automatically applying the plurality of operating parameters (358-1, 358-2, ..., 358-N) optimized for users with clinical hearing loss comprises applying the insertion gain.

9. The system of claim 7 or 8, wherein the process further comprises obtaining an audiogram (506); and
providing, in the hearing device (202), one or more speech enhancement features, wherein a strength of the speech enhancement provided by the speech enhancement features is adjustable,
wherein, when the subclinical hearing loss operating mode (304) is selected, the automatically applying the plurality of operating parameters (308-1, 308-2, ..., 308-N) optimized for users with subclinical hearing loss comprises adjusting the strength of one or more of the speech enhancement features to a predetermined strength independent from the audiogram (506), in particular a maximum strength;
and, when the clinical hearing loss operating mode (354) is selected, the automatically applying the plurality of operating parameters (358-1, 358-2, ..., 358-N) optimized for users with clinical hearing loss comprises adjusting the strength of one or more of the speech enhancement features to a strength depending on the audiogram.

10. The system of any one of claims 7 to 9, wherein the process further comprises
providing a plurality of clinical sound processing patterns attributed to different input sound classifications associated with a sound environment of a user, wherein the clinical sound processing patterns are optimized for sound processing in the clinical hearing loss operating mode;
providing a plurality of subclinical sound processing patterns attributed to the different input sound classifications, wherein the subclinical sound processing patterns are optimized for sound processing in the subclinical hearing loss operating mode;
selecting, when the clinical hearing loss operating mode (354) is selected, one or more of the clinical sound processing patterns, wherein the automatically applying the plurality of operating parameters (358-1, 358-2, ..., 358-N) optimized for users with clinical hearing loss comprises directing the hearing device (202) to process, when the hearing device determines the input sound classification during operation of the hearing device (202), the sound in accordance with the one or more selected clinical sound processing patterns attributed to the input sound classification determined by the hearing device (202);
and selecting, when the subclinical hearing loss operating mode (304) is selected, one or more of the subclinical sound processing patterns, wherein the automatically applying the plurality of operating parameters (308-1, 308-2, ..., 308-N) optimized for users with subclinical hearing loss comprises directing the hearing device (202) to process, when the hearing device determines the input sound classification during operation of the hearing device, the sound in accordance with the one or more selected subclinical sound processing patterns attributed to the input sound classification determined by the hearing device (202).

11. The system of any one of the preceding claims, wherein the automatically applying of the plurality of operating parameters (308-1, 308-2, ..., 308-N) to the hearing device (202) includes
selecting an input sound classification associated with sound in an environment of a user;
selecting, based on the input sound classification, a first subclinical hearing loss sound processing pattern from a plurality of subclinical hearing loss sound processing patterns; and
directing the hearing device (202) to process the sound in accordance with the first subclinical hearing loss sound processing pattern.

12. The system of claim 11, wherein the first subclinical hearing loss sound processing pattern is optimized for speech perception in noise for users with subclinical hearing loss.

13. The system of claim 11 or 12, wherein the automatically applying of the plurality of operating parameters (308-1, 308-2, ..., 308-N) to the hearing device (202) includes
selecting an additional input sound classification associated with sound in an environment of a user;
selecting, based on the additional input sound classification, a second subclinical hearing loss sound processing pattern from the plurality of subclinical hearing loss sound processing patterns; and
directing the hearing device to process the sound in accordance with the second subclinical hearing loss sound processing pattern instead of the first subclinical hearing loss sound processing pattern.

14. The system of claim 13, wherein the second subclinical hearing loss sound processing pattern is optimized for a context in which the user does not require speech enhancement.

15. A method comprising:
determining, by a hearing device management system, that a user of a hearing device (202) has subclinical hearing loss;
selecting, by the hearing device management system and based on the subclinical hearing loss of the user, a subclinical hearing loss operating mode (304) for the hearing device (202);
and automatically applying, by the hearing device management system to the hearing device (202) and based on the subclinical hearing loss operating mode, a plurality of operating parameters (308-1, 308-2, ..., 308-N) optimized for users with subclinical hearing loss.
